# EUROPEAN PATENT APPLICATION

(11) **EP 3 508 226 A1**
(43) Date of publication of application: **10.07.2019**
(21) Application number: 17846618.1
(22) Date of filing: 30.08.2017
(51) Int. Cl.: A61L 27/36, A61F 2/02

(54) **METHOD FOR FORMING CONNECTIVE TISSUE BODY**

(30) Priority: 31.08.2016 JP 2016169567
(71) Applicant: Biotube Co., Ltd., Nishiyodogawa-ku, Osaka-shi Osaka 5550011 (JP); National Cerebral and Cardiovascular Center, Suita-shi Osaka 565-8565 (JP)
(72) Inventor: NAKAYAMA, Yasuhide, Suita-shi Osaka 565-8565 (JP); MORIWAKI, Takeshi, Suita-shi Osaka 565-8565 (JP); OIE, Tomonori, Osaka-shi Osaka 555-0011 (JP)
(74) Representative: Westphal, Mussgnug & Partner Patentanwälte mbB
(86) International application number: PCT/JP2017/031276
(87) International publication number: WO 2018/043614

(57) **Abstract**

The present invention provides a method for forming a connective tissue body, which enables extending the ranges of design values in terms of shape, dimension, etc., of the connective tissue body. This method comprises: a fat treatment step (step S13) for removing fat contained in a connective tissue body, which is formed in an environment where a biological tissue material is present, from the interior of the connective tissue body while the connective tissue body is being set in a molding tool, and for causing the shape of the connective tissue body to follow the shape of the molding tool; and a bioinert solution treatment step (step S14) for immersing the connective tissue body, together with the molding tool, in a bioinert solution while the connective tissue body is being shaped so as to follow the shape of the molding tool after the fat treatment step.

## Description

The present invention relates to a connective tissue body forming method for forming a connective tissue body.

A self-defense function of the body mainly has properties in which a capsule configured of fibroblasts and an extracellular matrix encapsulates foreign substance(s). One type of regeneration medicine which is a medical treatment for regenerating lost tissues or organs with an artificial material embeds a tissue body forming device in a biological body as a foreign substance and then forms a biological body-derived connective tissue body from a living cell using the self-defense function described above (for example, refer to Patent Documents 1 to 3 listed below). At this time, the tissue body forming device used as the foreign substance includes two tissue body forming surfaces facing each other, and the connective tissue body is formed of a biological tissue material entering between the two tissue body forming surfaces (for example, refer to Patent Document 4 listed below).
Patent Document 1: Japanese Laid-Open Patent Publication No. 2007-312821
Patent Document 2: Japanese Laid-Open Patent Publication No. 2008-237896
Patent Document 3: Japanese Laid-Open Patent Publication No. 2010-094476
Patent Document 4: Japanese Laid-Open Patent Publication No. 2014-030598

A variety of biological body portions, such as a blood vessel, a cardiac valve, a cornea, and a tendon, require the application of the connective tissue body described above. For this reason, the shape or the dimension required for the connective tissue body varies according to a portion to which the connective tissue body is applied. The phenomenon in which the connective tissue body grows on a tissue body forming surface completely differs from a phenomenon in which a fluid merely spreads on the tissue body forming surface and is closely involved in the fact that the tissue body forming surface or the periphery thereof is recognized as a foreign substance. Therefore, it is not easy to obtain various shapes or dimensions with the tissue body forming device embedded in the biological body.

An object of the present invention is to provide a method for forming a connective tissue body that allows for broadening a range of a design value such as the shape or the dimension of a connective tissue body.

A method for forming a connective tissue body according to one aspect of the present invention includes a fat treatment step of removing fat contained in a connective tissue body, which is formed in an environment in which a biological tissue material exists and set in a molding die, from an inside of the connective tissue body and shaping the connective tissue body in conformance with a shape of the molding die. The method further includes a bioinert water treatment step of immersing the connective tissue body in bioinert water along with the molding die in a state in which the shape of the connective tissue body after the fat treatment step conforms to the shape of the molding die.

A connective tissue configuring the connective tissue body, in general, is a tissue that contains protein such as collagen or elastin, as a main component, and contains fat. According to the method described above, the fat treatment of removing the fat from the inside of the connective tissue body is performed thereby increasing a ratio of the extracellular matrix to all components configuring the connective tissue body. Then, the bioinert water treatment step of immersing the connective tissue body after the fat treatment in the bioinert water is performed. This forms the connective tissue body in conformance with the shape of the molding die and is applicable to regeneration medicine. As a result, it is possible to store a shape that conforms to the shape of the molding die in the connective tissue body and to broaden a range of a design value such as the shape or the dimension of the connective tissue body.

It will be apparent to those skilled in the art from this disclosure that in the technique of the present disclosure, a tissue corresponding to a connective tissue formed in a biological body also includes a tissue formed in an in-vitro environment. In addition, the biological tissue material is a substance necessary for forming a tissue derived from the biological body and, for example, includes animal cells such as fibroblasts cells, smooth muscle cells, ES cells, and iPS cells, an extracellular matrix including various proteins such as collagen or elastin, sugar such as hyaluronic acid, a cell growth factor of accelerating the cell growth or the cell specialization, and various bioactive substances existing in the biological body, such as cytokine. The biological tissue material may include materials derived from mammals such as human beings, dogs, cattle, pigs, goats, and sheep, birds, fishes, and other animals, and equivalent artificial materials. Then, the environment in which the biological tissue material exists, for example, is in the biological body in mammals such as human beings, dogs, cattle, pigs, goats, and sheep, birds, fishes, and other animals, under the skin of four limbs, the shoulders, the back, the abdomen, and the like, and an abdominal cavity. The environment in which the biological tissue material exists, for example, may be an artificial environment containing the biological tissue material.

In the method for forming a connective tissue body according to some aspects of the present invention, the removing the fat from the inside of the connective tissue body includes eluting the fat from the connective tissue body with an organic solvent that dissolves the fat by immersing the connective tissue body in the organic solvent in a state set in the molding die.

According to the method for forming a connective tissue body described above, the fat contained in the connective tissue body is eluted in the organic solvent thereby preventing the fat from being accumulated on the surface of the connective tissue body. This smoothly removes the fat from the connective tissue body.

In the method for forming a connective tissue body according to some aspects of the present invention, the removing the fat from the inside of the connective tissue body includes seeping the fat contained in the connective tissue body through a surface of the connective tissue body, by drying the connective tissue body in a state set in the molding die.

According to the method for forming a connective tissue body described above, the removing the fat from the inside of the connective tissue body can be performed by drying the connective tissue body. This allows for removal of moisture or the like, other than the fat, from the connective tissue body.

In the method for forming a connective tissue body according to some aspects of the present invention, the removing the fat from the inside of the connective tissue body includes eluting the fat from the connective tissue body in an organic solvent by immersing the connective tissue body in a state set in the molding die and drying the connective tissue body after being immersed in the organic solvent together with the molding die.

According to the method for forming a connective tissue body described above, it is also possible to remove the organic solvent used for eluting the fat, from the inside of the connective tissue body through the drying.

In the method for forming a connective tissue body according to some aspects of the present invention, the bioinert water treatment step keeps a density of proteins in an extracellular matrix of the connective tissue body higher than that before the fat treatment step.

According to the method for forming a connective tissue body described above, in the connective tissue body after the bioinert water treatment step, the density of the proteins in the extracellular matrix is kept high. Thus, the mechanical strength of the connective tissue body used in the regeneration medicine is higher than that before the fat treatment step.

In the method for forming a connective tissue body according to some aspects of the present invention, the fat treatment step thins the connective tissue body thin through drying, and the bioinert water treatment step sets a thickness of the connective tissue body to be greater than the thickness after the drying and less than the thickness before the fat treatment step.

According to the method for forming a connective tissue body described above, the thickness of the connective tissue body can be less than that before the fat treatment step. This can broaden a range of the thickness of the connective tissue body. As described above, the phenomenon that the connective tissue body grows on the tissue body forming surface is a phenomenon that completely differs from the fact that the fluid merely spreads on the tissue body forming surface and is closely involved in the fact that the tissue body forming surface or the periphery thereof is recognized as the foreign substance. In order to form a thin connective tissue body, for example, in the case of decreasing the thickness of a space for forming the connective tissue body, the space having a small thickness is hardly recognized as the foreign substance. Thus, it is difficult for the connective tissue to sufficiently form the connective tissue body that enters the space. Therefore, the molding of the connective tissue body having a small thickness is a particularly outstanding effect in broadening the range of the design value on the structure of the connective tissue body. Then, the connective tissue body is molded such that the thickness of the connective tissue body decreases in a state where the density of the proteins increases in the extracellular matrix of the connective tissue body. Thus, the thickness of the connective tissue body can be decreased while limiting decreases in the mechanical strength of the connective tissue body.

In the method for forming a connective tissue body according to some aspects of the present invention, the fat treatment step elutes the fat from the connective tissue body in the organic solvent by immersing the connective tissue body in the organic solvent in a state in which the molding die having a smaller diameter than a tubular portion of the connective tissue body is internally inserted into the connective tissue body to closely attach the tubular portion to the molding die through the drying.

According to the method for forming a connective tissue body described above, the inner diameter of the tubular portion of the connective tissue body can be decreased. Thus, the range of the design value can be broadened with respect to the inner diameter of the tubular portion of the connective tissue body. As described above, in order to form the connective tissue body including the tubular portion having a small diameter, for example, in a case where a cylindrical material having a small diameter is embedded in the biological body, the cylindrical material having a small diameter is hardly recognized as the foreign substance. Thus, it would be difficult to form the connective tissue body in a part of a surface of the cylindrical material. Therefore, reducing the inner diameter of the tubular portion is a particularly outstanding effect in broadening the range of the design value on the structure of the connective tissue body. Furthermore, the inner diameter of the tubular portion decreases in a state where the density of the proteins increases in the extracellular matrix of the connective tissue body. Thus, the inner diameter of the tubular portion can be decreased while limiting decreases in the mechanical strength of the connective tissue body.

In the method for forming a connective tissue body according to some aspects of the present invention, the fat treatment step includes eluting the fat from the connective tissue body in the organic solvent by immersing the connective tissue body in the organic solvent after the molding die, which extends in a direction differing from an extension direction of a tubular portion of the connective tissue body, is internally inserted into the connective tissue body and an extension direction of the connective tissue body conforms to an extension direction of the molding die.

According to the method for forming a connective tissue body described above, the extension direction of the tubular portion in the connective tissue body can be molded in the extension direction of the molding die. Thus, the degree of freedom can be increased for the extension direction of the tubular portion of the connective tissue body. When, for example, embedding the cylindrical material in a desired direction in the biological body to form the connective tissue body including the tubular portion extending in a desired direction, it is necessary to ensure a space occupied by the cylindrical material in the biological body. When it is difficult to ensure the space occupied by the cylindrical material in the biological body or the like, a load on the biological body increases forcing the surface of the biological body to greatly bulge. Therefore, changing the extension direction of the tubular portion is a particularly outstanding effect in broadening the range of the design value on the structure of the connective tissue body.

According to the method for forming a connective tissue body of one or more aspects of the present invention, it is possible to broaden the range of the design value on the structure such as the shape or the dimension of the connective tissue body.
Fig. 1 is a flowchart illustrating a flow of steps of a method for forming a connective tissue body in one embodiment.
Fig. 2 is a graph illustrating a relationship between a thickness of the connective tissue body and a time for a fat treatment.
Figs. 3A to 3C are process charts illustrating an exemplified step of decreasing a thickness of a tubular portion.
Figs. 4A to 4D are process charts illustrating an exemplified step of decreasing a diameter of the tubular portion.
Figs. 5A to 5D are process charts illustrating an exemplified step of decreasing a diameter of a part of the tubular portion.
Figs. 6A to 6D are process charts illustrating an exemplified step of deforming a side shape of the tubular portion.
Figs. 7Ato 7C are process charts illustrating an exemplified step of changing an extension direction of the tubular portion.
Figs. 8A to 8C are process charts illustrating an exemplified step of forming a sheet-shaped connective tissue body.

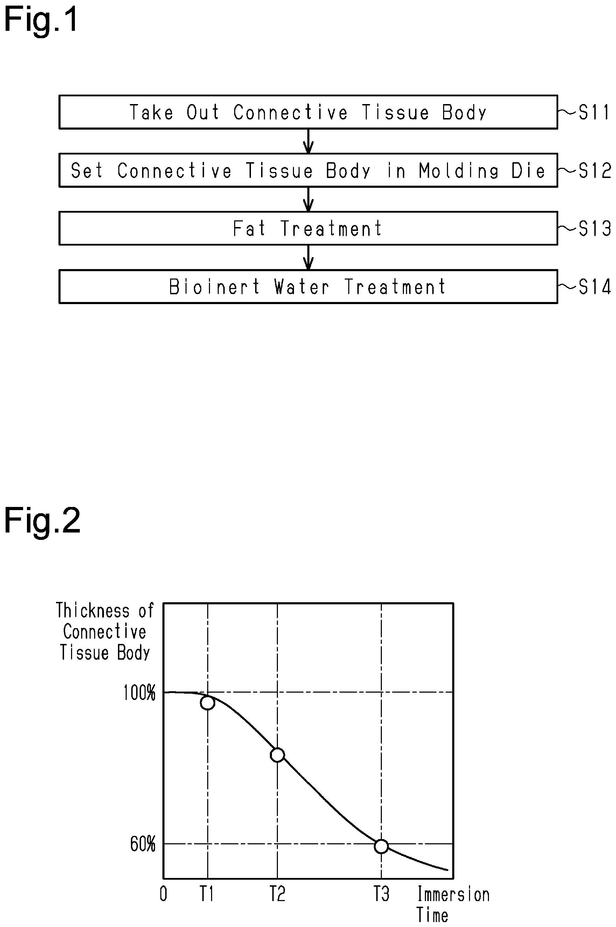

One embodiment of a method for forming a connective tissue body will now be described with reference to Figs. 1 to 8.

As illustrated in Fig. 1, the method for forming a connective tissue body includes a step of taking out the connective tissue body (step S11), and a step of setting the connective tissue body in a molding die (step S12). The method for forming the connective tissue body further includes a step of performing a fat treatment (step S13), and a step of performing a bioinert water treatment (step S14).

### [Taking-out Step]

The step of taking out the connective tissue body takes out the connective tissue body formed in an environment where a biological tissue material exists from the environment in which the biological tissue material exists. The environment in which the biological tissue material exists, for example, is in a biological body in mammals such as human beings, dogs, cattle, pigs, goats, and sheep, birds, fishes, and other animals, under the skin of four limbs, the shoulders, the back, the abdomen, and the like, and an abdominal cavity. The environment in which the biological tissue material exists, for example, may be an artificial environment containing he biological tissue material.

In an exemplified step of forming the connective tissue body in the environment where the biological tissue material exists, first, a tissue body forming device for forming the connective tissue body, is embedded in the environment where the biological tissue material exists. In a case where the tissue body forming device is embedded in the biological body, first, minimum incision is performed with respect to the biological body, under sufficient anesthesia. Then, the tissue body forming device is embedded, and then, the cut is sutured. In the tissue body forming device embedded in the environment where the biological tissue material exists, the connective tissue body is formed in the space of the tissue body forming device recognized as a foreign substance, or on a surface of the tissue body forming device. After a predetermined embedding period, which is a period in which the connective tissue body is formed, elapses, the tissue body forming device embedded in the environment where the biological tissue material exists is taken out from the environment. In a case where the tissue body forming device is taken out from the biological body, first, minimum incision is performed with respect to the biological body, under sufficient anesthesia. Then, the tissue body forming device is taken out. Then, the cut is sutured.

For example, the tissue body forming device is in the shape of a double tube extending in one direction, and an outer surface of an outer tube includes a hole connected to a gap between an inner tube and the outer tube, on. Then, in the tissue body forming device embedded in the environment where the biological tissue material exists, a connective tissue enters the gap between the inner tube and the outer tube from the outer surface of the outer tube, and a tubular connective tissue body for filling the gap is formed by the connective tissue entering the gap.

In addition, for example, the tissue body forming device is in the shape of a plate of two layers facing each other, and an upper surface of an upper plate includes a hole connected a gap between the upper plate and a lower plate. Then, in the tissue body forming device embedded in the environment where the biological tissue material exists, the connective tissue enters the gap between the upper plate and the lower plate from the upper surface of the upper plate, and a sheet-shaped connective tissue body for filling the gap is formed by the connective tissue entering the gap.

In addition, for example, the tissue body forming device is a structure including a tissue body forming surface that is a surface on which the connective tissue body is formed on the outermost surface. Then, in the tissue body forming device embedded in the environment where the biological tissue material exists, the connective tissue body having a surface shape that conforms to the shape of the tissue body forming surface grows on the tissue body forming surface.

In regeneration medicine, a portion in which the application of the connective tissue body is required, is various such as a blood vessel, a cardiac valve, a cornea, and a tendon. For this reason, the shape or the dimension required for the connective tissue body varies according to a portion to which the connective tissue body is applied. A phenomenon that the connective tissue body grows in the tissue body forming device is a phenomenon closely involved in the fact that the gap, the tissue body forming surface, or the like is recognized as the foreign substance. Therefore, in the tissue body forming device embedded in the environment where the biological tissue material exists, a configuration before being molded, such as the shape of a tube or a plate, is applied to the connective tissue body, but it is difficult to directly give the detailed shape or dimension that can be applied to the regeneration medicine.

### [Setting Step]

The step of setting the connective tissue body in the molding die sets the connective tissue body that is taken out from the environment in which the biological tissue material exists in the molding die. The molding die, for example, has elasticity lower than that of the connective tissue body to be set and has plasticity sufficiently higher than that of the connective tissue body. A material configuring the molding die has compatibility with the biological body, such as non-toxic properties and bioinert properties, and is an inorganic compound including an alloy such as stainless steel, ceramics, or an organic compound including a polymer such as silicon. The shape of the molding die conforms to the shape of the portion in which the application of the connective tissue body is required.

For example, in a case where the portion in which the application of the connective tissue body is required is a blood vessel, the molding die is a linear mold extending in an extension direction of the blood vessel and internally inserted into the connective tissue body, in which an inner diameter of the blood vessel is set to an outer diameter. In addition, for example, in a case where the portion in which the application of the connective tissue body is required is the blood vessel, the molding die is a mesh-shaped mold embedded in the connective tissue body, which is a tubular member having a shape that conforms to the shape of the blood vessel.

In addition, for example, in a case where the portion in which the application of the connective tissue body is required, is a cardiac valve, the molding die is a linear mold to be embedded in the connective tissue body, of which the shape conforms to the shape of the cardiac valve. In addition, for example, in a case where the portion in which the application of the connective tissue body is required is a cornea, the molding die is a plate-shaped die fixing an outer edge of a sheet-shaped connective tissue body to a member of which a surface shape conforms to a surface shape of the cornea. In addition, for example, in a case where the portion in which the application of the connective tissue body is required is a tendon, the molding die is a tubular mold externally inserted into the connective tissue body that linearly extends and has the shape of a tube extending in an extension direction of the tendon.

For example, setting the connective tissue body in the molding die fits the molding die into the connective tissue body and fits a linear or cylindrical molding die into a tube of a tubular connective tissue body or internally inserts or loosely inserts a linear molding die into the tube of the tubular connective tissue body.

In addition, for example, setting the connective tissue body in the molding die embeds the molding die in the connective tissue body and inserts a linear molding die into a tubular or sheet-shaped connective tissue body or embeds a mesh-shaped molding die having flexibility in the tubular or sheet-shaped connective tissue body. It will be apparent to those skilled in the art from this disclosure that embedding the molding die in the connective tissue body may embed a mesh-shaped molding die in the environment where the biological tissue material exists to form the connective tissue body in the environment described above such that the molding die is embedded in the connective tissue body. In other words, in a case where the tissue body forming device used for forming the connective tissue body functions as the molding die, setting the connective tissue body in the molding die may be combined with the step of taking out the connective tissue body.

It will be apparent to those skilled in the art from this disclosure that the shape of the connective tissue body set in the molding die, for example, may be a shape ablating a part of the connective tissue body that is taken out from the environment in which the biological tissue material exists or may be a shape differing from the connective tissue body that is taken out from the environment in which the biological tissue material exists. For example, the shape of the connective tissue body set in the molding die may be a shape in which a part of the connective tissue body is ablated or may be a part of the connective tissue body formed by the tissue body forming device in order to separate the connective tissue body from the tissue body forming device.

### [Fat Treatment Step]

The step of performing the fat treatment removes fat from the inside of the connective tissue body. An exemplified step of performing the fat treatment elutes fat contained inside the connective tissue body in an organic solvent by immersing the connective tissue body in the organic solvent in a state set in the molding die. Another exemplified step of performing the fat treatment seeps the fat contained inside the connective tissue body through a surface of the connective tissue body by drying the connective tissue body in a state set in the molding die. The step of performing the fat treatment may also be a combination of the above. An exemplified combination elutes the fat contained inside the connective tissue body in the organic solvent by immersing the connective tissue body in the organic solvent in a state set in the molding die and then drying the organic solvent contained inside the connective tissue body. Another exemplified combination seeps the fat contained inside the connective tissue body, to seep through the surface of the connective tissue body, by drying the connective tissue body in a state set in the molding die and then removes the fat from the surface of the connective tissue body by immersing the connective tissue body in the organic solvent. In a fat treatment step, the connective tissue body is immersed in the organic solvent or dried together with the molding die, such that the shape of the connective tissue body conforms to the shape of the molding die.

The organic solvent used in the fat treatment step is a liquid that gives priority to the elution of the fat over proteins such as collagen or elastin in an extracellular matrix. In addition, the organic solvent used in the fat treatment step is a liquid that does not allow the decomposition or the cross-linkage of the proteins in the extracellular matrix to progress unlike an aqueous solution of formaldehyde or the like that allows the cross-linkage of the protein to progress. Examples of the organic solvent used in the fat treatment step, include alcohols such as methanol, ethanol, and isopropyl alcohol, polyhydric alcohols such as ethylene glycol, a pyrrolidone-based solvent, and acetones.

The connective tissue body is immersed in the organic solvent over a time in which the fat contained in the connective tissue body can be eluted in the organic solvent. The connective tissue body is immersed in the organic solvent over a time in which a suitable change is performed according to the shape or the dimension of the connective tissue body. For example, when the thickness of the connective tissue body is greater than or equal to 0.5 mm and less than or equal to 5 mm, the time for immersing the connective tissue body in ethanol is longer than or equal to 10 minutes and shorter than or equal to 60 minutes.

A whitish milky color of the connective tissue body during the fat treatment before being immersed in the organic solvent is lost along with the elution of the fat, and the connective tissue body becomes closer to a state of being colorless and transparent as the amount of fat to be eluted increases. For this reason, it is possible to manage an immersion time of the organic solvent on the basis of a management value that is at least one of the chromaticness, the lightness, and the weight of the connective tissue body during the fat treatment. For example, the management value when the amount of fat contained in the connective tissue body is a desired value is set as a target value. Then, when the management value of the connective tissue body reaches the target value, the immersion in the organic solvent is ended. According to such management of the immersion time, it is possible to increase reproducibility with respect to the state of the connective tissue body after the immersion. In addition, it is also possible to confirm the state of the connective tissue body during the immersion without the need to contact the connective tissue body.

The fat treatment of eluting the fat from the inside of the connective tissue body is performed thereby increasing a ratio of proteins such as collagen or elastin, to all components configuring the connective tissue body. A possibility that the fat contained in the connective tissue body is malformed is higher than a possibility that the protein is malformed. For this reason, the immersing in the organic solvent is performed so that the remaining malformed fat is reduced in the connective tissue body after the fat treatment. This increases the ratio of the proteins to all components configuring the connective tissue body originally required as the connective tissue body. The connective tissue body in which the ratio of the proteins increases is deformed to adhere to the molding die, and a tensile force acting on the connective tissue body increases. Then, the density of the proteins in the connective tissue body increases, as the elution of the fat progresses. Thus, a shape that conforms to the shape of the molding die is stored in the connective tissue body. In addition, in the connective tissue body where the shape is stored, the cross-linkage of the protein does not progress, and the fixation in the connective tissue body does not progress. Thus, in the subsequent bioinert water treatment, it is possible to contain bioinert water in the connective tissue body and to separate the molding die from the connective tissue body.

In a drying treatment performed in the fat treatment step, the connective tissue body is dried in an atmosphere of the drying treatment such that the fat contained inside the connective tissue body set in the molding die seeps through the surface of the connective tissue body.

A pressure in a drying environment in the fat treatment step is lower than the vapor pressure of the moisture contained in the connective tissue body. A temperature in the drying environment is a temperature at which the decomposition or the cross-linkage of collagen contained in the connective tissue body does not progress. The drying environment, for example, may be in the atmospheric air managed in a standard state or may be in an atmosphere of inert gas for preventing the connective tissue body from being malformed.

It will be apparent to those skilled in the art from this disclosure that in a case where the material configuring the molding die is a material having plasticity such as a metal, for example, the molding die immersed in the organic solvent may be deformed in the middle of the treatment. In addition, after deforming the molding die immersed in the organic solvent, the drying treatment of the connective tissue body may be performed while keeping the deformed molding die in such a state. Alternatively, the molding die in the drying treatment may be deformed in the middle of the treatment. According to a method in which the molding die is deformed, in the fat treatment, a large surface area of the connective tissue body is ensured. Thus, it is possible to accelerate the elution of the fat and accelerate the seeping of the fat.

The amount of moisture of the connective tissue body before the drying treatment is approximately greater than or equal to 80%, and the connective tissue body is dried over a time in which the amount of moisture of the connective tissue body before the drying treatment, for example, is less than or equal to 10%. The connective tissue body is dried over a time when the fat is capable of seeping through the surface of the connective tissue body and is suitably changed according to the thickness of the connective tissue body before the drying treatment, a shape required for the connective tissue body, or the like. It will be apparent to those skilled in the art from this disclosure that in a case where the connective tissue body is immersed in the organic solvent, before the drying treatment, the connective tissue body is dried over a time in which the organic solvent contained in the connective tissue body can be removed. For example, if the organic solvent is ethanol, the thickness of the connective tissue body before the drying treatment is greater than or equal to 0.5 mm and less than or equal to 5 mm and the drying environment is in the atmospheric air managed in the standard state, a removal time for removing ethanol from the connective tissue body is longer than or equal to 1 hour and shorter than or equal to 6 hours. The time for drying the connective tissue body becomes longer as the thickness of the connective tissue body before the drying treatment increases.

The state before the drying in which the connective tissue body is colorless and transparent during the drying treatment is lost as the organic solvent contained in the connective tissue body decreases and the amount of moisture contained in the connective tissue body decreases such that the connective tissue body becomes closer to a brownish yellow color. For this reason, it is possible to manage a drying time of the connective tissue body on the basis of the management value that is at least one of the chromaticness, the lightness, and the weight of the connective tissue body during the drying treatment. For example, the management value for when the amount of organic solvent contained in the connective tissue body or the amount of moisture contained in the connective tissue body is a desired value that is set as a target value. Then, when the management value in the connective tissue body reaches the target value, the drying treatment is ended. According to the management of the drying time, as described above, it is possible to increase reproducibility with respect to the state of the connective tissue body after the drying treatment. In addition, it is possible to confirm the state of the connective tissue body during the drying treatment without the need to contact the connective tissue body.

The fat treatment that seeps the fat from the inside of the connective tissue body is performed thereby increasing the ratio of the proteins such as collagen or elastin to all components configuring the connective tissue body. For this reason, the drying described above is performed thereby reducing the remaining the malformed fat in the connective tissue body after the fat treatment. This increases the ratio of the proteins to all components configuring the connective tissue body originally required as the connective tissue body. The connective tissue body in which the ratio of the proteins increases is deformed to adhere to the molding die, and a tensile force acting on the connective tissue body increases. Then, the density of the proteins increases as the seeping of the fat progresses, and the shape that conforms to the shape of the molding die is stored in the connective tissue body.

It will be apparent to those skilled in the art from this disclosure that in the fat treatment described above, the fat or the moisture is removed from the surface of the connective tissue body before the inside of the connective tissue body. At this time, there is a difference between a contraction degree on the surface of the connective tissue body and a contraction degree on the inside of the connective tissue body. There is a case where such a difference between the contraction degrees causes a deviation of the shape of the connective tissue body from the shape of the molding die. For this reason, it is preferable that the deviation between the shape of the connective tissue body and the shape of the molding die be limited by using a fixing tool for matching the shape of the connective tissue body to the shape molding die, such as a pin, while the fat treatment is performed.

### [Bioinert Water Treatment Step]

The step of performing the bioinert water treatment contains the bioinert water in the connective tissue body after the fat treatment. The step of performing the bioinert water treatment immerses the connective tissue body after the fat treatment in the bioinert water together with the molding die. The step of performing the bioinert water treatment has the connective tissue body contain the bioinert water that is required for application to the connective tissue body and required for the preservation of the connective tissue body. In addition, when containing the organic solvent in the connective tissue body in the fat treatment step, the step of performing the bioinert water treatment substitutes the organic solvent remaining in the connective tissue body with the bioinert water. The bioinert water used in a bioinert water treatment step is saline, a dextrose solution, a Ringer solution, a composite electrolyte solution, or the like.

The connective tissue body is immersed in the bioinert water over a time in which the bioinert water can be contained in the connective tissue body. In addition, the connective tissue body is immersed in the bioinert water over a time that is suitably changed according to a period in which the connective tissue body is preserved.

The colorless and transparent or brownish yellow color of the connective tissue body immersed in the bioinert water before being immersed in the bioinert water is lost by the bioinert water, and the connective tissue body becomes closer to a whitish milky color as the amount of bioinert water increases. For this reason, it is possible to manage an immersion time of the bioinert water on the basis of the management value that is at least one of the chromaticness, the lightness, and the weight of the connective tissue body immersed in the bioinert water. For example, the management value when the amount of bioinert water contained in the connective tissue body is a desired value is set as a target value. Then, when the management value in the connective tissue body reaches the target value, the bioinert water treatment is ended. According to the management of the immersion time, as described above, it is possible to increase reproducibility with respect to the state of the connective tissue body after the bioinert water treatment. In addition, it is possible to confirm the state of the connective tissue body during the bioinert water treatment, without the need to contact the connective tissue body.

When the drying treatment is performed in the fat treatment step and the bioinert water treatment of containing the bioinert water in the connective tissue body, is performed, the ratio of the proteins such as collagen or elastin to all components configuring the connective tissue body is lower than that immediately after the drying. In addition, the thickness of the connective tissue body is greater than that immediately after the drying treatment step. At this time, the large thickness of the connective tissue body is less than or equal to the thickness of the connective tissue body before the fat treatment. In addition, even in the connective tissue body having a large thickness, the shape that conforms to the shape of the molding die, is stored for a considerable time. As a result, it is possible to store the shape that conforms to the shape of the molding die in the connective tissue body and to broaden a range of a design value on the structure of the connective tissue body.

As illustrated in Fig. 2, a change amount in the thickness of the connective tissue body after the bioinert water treatment is changed according to the amount of fat removed from the connective tissue body. For example, when the connective tissue body is immersed in the organic solvent and then dried in the fat treatment step, a change amount in the thickness of the connective tissue body after the bioinert water treatment is changed according to the immersion time when the connective tissue body is immersed in the organic solvent. If the immersion time when the connective tissue body is immersed in the organic solvent is shorter than or equal to a retention time T1, the thickness of the connective tissue body after the bioinert water treatment is maintained at approximately the thickness before the fat treatment. The retention time T1 is changed according to the thickness of the connective tissue body before the fat treatment. In a case where the thickness of the connective tissue body before the fat treatment is 1 mm, the retention time T1, for example, is 10 minutes. In a case where the thickness of the connective tissue body before the fat treatment is 10 mm, the retention time T1, for example, is 60 minutes.

When the time in the connective tissue body is immersed in the organic solvent is longer than the retention time T1, the thickness of the connective tissue body after the bioinert water treatment is less than the thickness before the fat treatment as the time when the connective tissue body is immersed in the organic solvent, increases. For example, in a case where the thickness of the connective tissue body before the fat treatment is 1 mm and an immersion time T2 is 30 minutes, the thickness of the connective tissue body after the bioinert water treatment, is approximately 80% of the thickness before the fat treatment. Further, in a case where the thickness of the connective tissue body before the fat treatment is 1 mm, and an immersion time T3 is 60 minutes, the thickness of the connective tissue body after the bioinert water treatment, decreases up to 60% of the thickness before the fat treatment. It will be apparent to those skilled in the art from this disclosure that when the time when the connective tissue body is immersed in the organic solvent, is shorter than or equal to the retention time T1, an example of a collagen density in the connective tissue body after the bioinert water treatment, is 200 mg/cm³. When the time when the connective tissue body is immersed in the organic solvent, is the immersion time T3, an example of the collagen density in the connective tissue body after the bioinert water treatment is 400 mg/cm³.

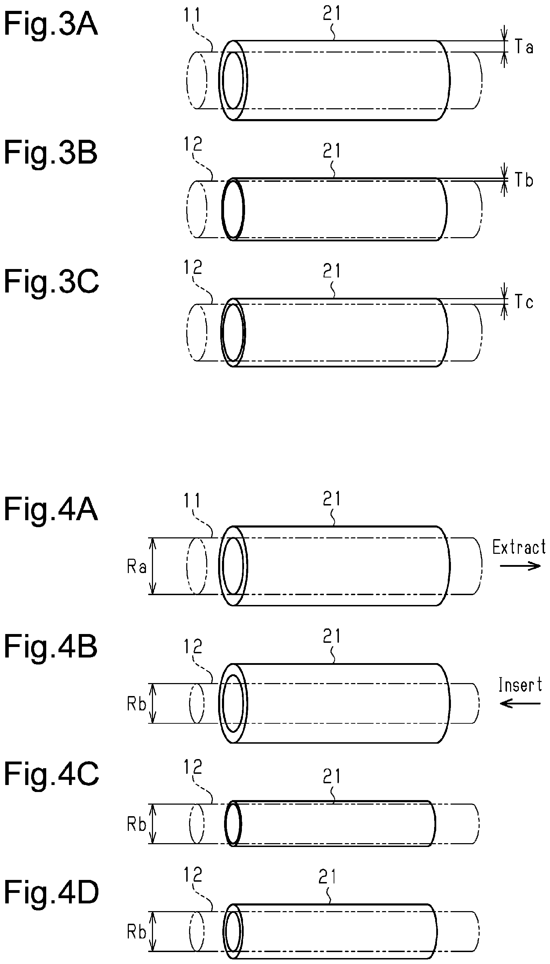

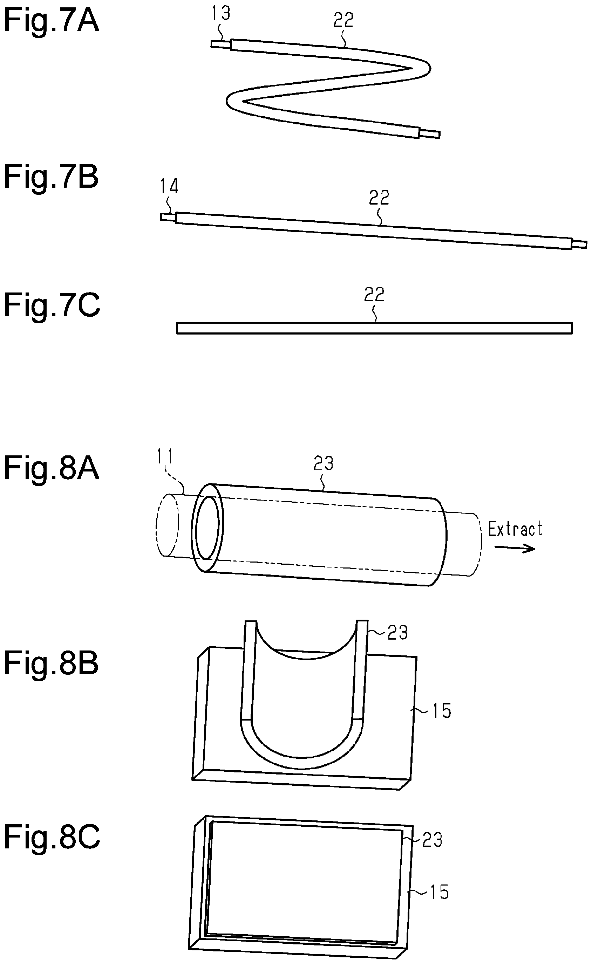

Next, an example of the shape and the dimension of the connective tissue body in each step of the method for forming a connective tissue body will be described. It will be apparent to those skilled in the art from this disclosure that in each of Figs. 3 to 7, an example will be described in which a tubular connective tissue body is formed by using the tissue body forming device, and a cylindrical molding die is set in a tube of the tubular connective tissue body. In each of Figs. 3 to 7, the shapes of the molding die set in the connective tissue body differ from each other. In addition, in Fig. 8, an example will be described in which the tubular connective tissue body is formed by the tissue body forming device, and a plate-shaped molding die is set in the opened connective tissue body. In addition, in each example illustrated in Figs. 3 to 6, in the fat treatment step described above, the treatment of immersing the connective tissue body in the organic solvent and the subsequent drying treatment are performed as the fat treatment step. In addition, in an example illustrated in Fig. 7, the treatment of immersing the connective tissue body in the organic solvent is performed as the fat treatment step. In addition, in an example illustrated in Fig. 8, the treatment of drying the connective tissue body is performed as the fat treatment step.

As illustrated in Fig. 3A, a cylindrical member 11 is taken out from the environment in which the biological tissue exists. A connective tissue body 21 is positioned on the outer surface of the taken cylindrical member 11. The cylindrical member 11 functions as the tissue body forming device. In the setting step, the fat treatment step, and the bioinert water treatment step that are described above, the cylindrical member 11 functions as a cylindrical die 12 that is an example of the molding die. That is, the connective tissue body 21 taken out from the environment in which the biological tissue exists is taken out from the environment, in a state set in the molding die. Then, the connective tissue body 21, which is set in the cylindrical die 12, is immersed in the organic solvent such as ethanol together with the cylindrical die 12 thereby eluting the fat contained in the connective tissue body 21 in the organic solvent. The immersion time in the solvent immersion treatment, for example, is the immersion time T3 described in Fig. 2. When the solvent immersion treatment is ended, the connective tissue body 21 that is whitish before the solvent immersion treatment becomes generally colorless and transparent.

As illustrated in Fig. 3B, the connective tissue body 21 after the solvent immersion treatment, is left to stand in the drying environment as with in the atmosphere of inert gas or the like. The organic solvent, the moisture, or the like contained in the connective tissue body 21 is removed from the connective tissue body 21 in the drying environment. A thickness Tb of the connective tissue body 21 is considerably less than a thickness Ta when the solvent immersion treatment is ended. When the drying treatment is ended, the thickness Tb of the connective tissue body 21, for example, is decreased to less than or equal to 30% of the thickness Ta before the drying treatment is started.

The connective tissue body 21 is deformed such that an inner surface of the connective tissue body 21 is adheres to an outer surface of the cylindrical die 12 as the connective tissue body 21 dries. Then, the density of the proteins in the connective tissue body 21 increases, and a tubular shape that conforms to the shape of the outer surface of the cylindrical die 12 is stored in the connective tissue body 21. At this time, the connective tissue body 21 that is colorless and transparent before the drying treatment is changed to a brownish yellow connective tissue body 21.

As illustrated in Fig. 3C, the connective tissue body 21 after the fat treatment, is immersed in the bioinert water such as saline, together with the cylindrical die 12. Then, the bioinert water infiltrates into the connective tissue body 21, and a thickness Tc of the connective tissue body 21, is slightly greater than the thickness Tb when the drying treatment is ended. When the bioinert water treatment is ended, the thickness Tc of the connective tissue body 21, is less than or equal to 60% of the thickness Ta when the solvent immersion treatment is ended, and is less than the thickness of the connective tissue body before the fat treatment.

At this time, the connective tissue body 21, which is brownish yellow before the bioinert water treatment, is changed to a whitish connective tissue body 21. Then, even in the connective tissue body 21 having a large thickness, the shape that conforms to the shape of the outer surface of the cylindrical die 12 is stored for a considerable time, and the thickness Ta of the connective tissue body 21 is changed to the thickness Tc of the connective tissue body 21. As a result, it is possible to store the shape that conforms to the shape of the outer surface in the cylindrical die 12 in the connective tissue body 21 to reduce the thickness of the connective tissue body 21 and to broaden a range for the design value on the structure of the connective tissue body 21. In addition, the thickness Ta of the connective tissue body 21 is changed to the thickness Tc of the connective tissue body 21, and most of the proteins in the connective tissue body 21 are also kept in the connective tissue body 21. Therefore, in the connective tissue body 21, the density of the proteins increases.

It will be apparent to those skilled in the art from this disclosure that a phenomenon that the connective tissue body 21 grows is a phenomenon that completely differs from the fact that a fluid merely spreads on a surface on which the connective tissue body 21 is formed and closely involved in the fact that the surface on which the connective tissue body 21 is formed or its periphery is recognized as the foreign substance. In order to form the connective tissue body 21 having a small thickness, for example, in the case of decreasing the thickness of the space for forming the connective tissue body 21, the space having a small thickness is hardly recognized as the foreign substance. Thus, it is difficult for sufficient connective tissue to enter the space and form the connective tissue body 21. Therefore, molding the connective tissue body 21 with a small thickness is a particularly outstanding effect broadening the range with respect to the design value on the structure of the connective tissue body.

In addition, the small thickness Tc of the connective tissue body 21 is realized in a state where the density of the proteins in the connective tissue body 21 is increased. For this reason, it is possible to decrease the thickness of the connective tissue body 21 while limiting decreases in the mechanical strength of the connective tissue body 21.

It will be apparent to those skilled in the art from this disclosure that in an example illustrated in Fig. 3, for example, it is also possible to use an elliptical cylindrical member or a polygonal columnar member as the molding die by using an elliptical tubular or sectional polygonal connective tissue body 21.

As illustrated in Fig. 4A, the cylindrical member 11 is taken out from the environment in which the biological tissue exists. The connective tissue body 21 is positioned on the outer surface of the taken cylindrical member 11. The cylindrical member 11 has an outer diameter Ra and functions as the tissue body forming device. Then, the cylindrical member 11 is extracted from the tube of the connective tissue body 21, and a tubular connective tissue body 21 having the outer diameter Ra as an inner diameter is formed.

As illustrated in Fig. 4B, the cylindrical die 12 functioning as the molding die is set in the connective tissue body 21 that is an example of a tubular portion. That is, the cylindrical die 12 having an outer diameter Rb less than the outer diameter Ra is loosely inserted into the tube of the connective tissue body 21. The connective tissue body 21 set in the cylindrical die 12 is immersed in the organic solvent such as ethanol together with the cylindrical die 12. The fat contained in the connective tissue body 21 is eluted in the organic solvent from an outer circumferential surface of the connective tissue body 21 and an inner circumferential surface of the connective tissue body 21. The immersion time in the solvent immersion treatment, for example, is the immersion time T3 described in Fig. 2. When the solvent immersion treatment is ended, the connective tissue body 21 that is whitish before the solvent immersion treatment becomes generally colorless and transparent.

As illustrated in Fig. 4C, the connective tissue body 21 after the solvent immersion treatment is left to stand in the drying environment as with in the atmosphere of inert gas, or the like. Then, the organic solvent, the moisture, or the like is removed from the inside of the connective tissue body 21 as the connective tissue body 21 dries, and the connective tissue body 21 is deformed such that the connective tissue body 21 adheres to the outer surface of the cylindrical die 12. Accordingly, an inner diameter of the connective tissue body 21 is considerably less than an inner diameter when the solvent immersion treatment is ended. When the drying treatment is ended, the inner diameter of the connective tissue body 21 is equal to the outer diameter Rb of the cylindrical die 12. Then, the density of the proteins in the connective tissue body 21 increases, and a tubular shape that conforms to the shape of the outer surface of the cylindrical die 12 is stored in the connective tissue body 21. At this time, the connective tissue body 21 that is colorless and transparent before the drying treatment is changed to the brownish yellow connective tissue body 21.

As illustrated in Fig. 4D, after the fat treatment, the connective tissue body 21 is immersed in the bioinert water such as saline together with the cylindrical die 12. Then, the bioinert water infiltrates the connective tissue body 21, and the thickness of the connective tissue body 21 becomes slightly greater than a thickness when the drying treatment is ended. During such a period, the inner diameter of the connective tissue body 21 is equal to the outer diameter Rb of the cylindrical die 12. This keeps the size smaller than the outer diameter Ra before the drying treatment. That is, even in the connective tissue body 21 having a large thickness, the shape that conforms to the outer surface of the cylindrical die 12 is stored for a considerable time, and the inner diameter of the connective tissue body 21 decreases to the outer diameter Rb. As a result, it is possible to store the shape that conforms to the shape of the outer surface of the cylindrical die 12 in the connective tissue body 21 to decrease the inner diameter of the connective tissue body 21 and to broaden the range with respect to the design value on the structure of the connective tissue body 21. In addition, as with the example described in Fig. 3, most of the proteins in the connective tissue body 21 are maintained in the connective tissue body 21. Therefore, in the connective tissue body 21, the density of the proteins increases.

It will be apparent to those skilled in the art from this disclosure that in an example illustrated in Fig. 4, for example, it is possible to use the cylindrical die 12 as the molding die, by using an elliptical tubular or sectional polygonal connective tissue body 21. At this time, it is also possible to bring the inner surface of the connective tissue body 21, into surface contact with a part of the outer surface of the cylindrical die 12. In the elliptical tubular connective tissue body 21, for example, it is possible to increase the thickness of the connective tissue body 21 after the bioinert water treatment compared to a tubular connective tissue body having a short diameter of an elliptical tube as the inner diameter. In a method for forming the connective tissue body 21 in a state where a plurality of circular tubes are arranged and a method for forming the connective tissue body 21 in a state where elliptical tubes are arranged in a short diameter direction of the elliptical tube, a length required in the short diameter direction at the time of forming the connective tissue body 21 can be equalized in the environment in which the biological tissue exists. That is, it is possible to increase the thickness of the connective tissue body 21 while equalizing the size of the space for forming the connective tissue body 21 in the short diameter direction.

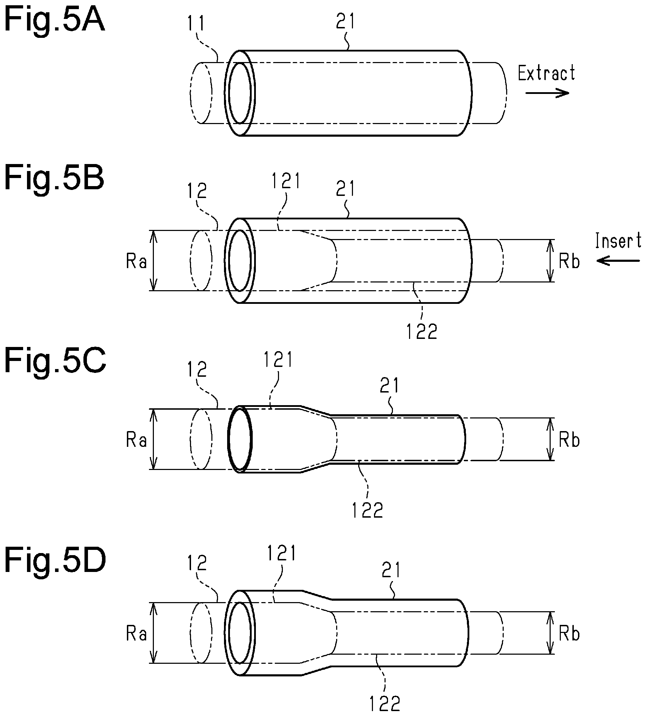

As illustrated in Fig. 5A, the cylindrical member 11 is taken out from the environment in which the biological tissue exists. The connective tissue body 21 is positioned on the outer surface of the taken cylindrical member 11. The cylindrical member 11 functions as the tissue body forming device having the same outer diameter along the extension direction. Then, the cylindrical member 11 is extracted from the tube of the connective tissue body 21.

As illustrated in Fig. 5B, the cylindrical die 12 functioning as the molding die is set in the connective tissue body 21 that is an example of the tubular portion. The cylindrical die 12 is in the shape of a two-stage cylinder including a large-diameter portion 121, which has the outer diameter Ra, and a small-diameter portion 122, which has the outer diameter Rb that is smaller than the outer diameter Ra. The large-diameter portion 121 of the cylindrical die 12 is fitted into the tube of the connective tissue body 21, and the small-diameter portion 122 of the cylindrical die 12 is loosely inserted into the tube of the connective tissue body 21. The connective tissue body 21 set in the cylindrical die 12 is immersed in the organic solvent such as ethanol together with the cylindrical die 12. A part of the fat contained in the connective tissue body 21, is eluted in the organic solvent from the outer circumferential surface of the connective tissue body 21 and the inner circumferential surface of the connective tissue body 21. The immersion time in the solvent immersion treatment, for example, is the retention time T1 described in Fig. 2. When the solvent immersion treatment is ended, the connective tissue body 21, which is whitish before the solvent immersion treatment, becomes closer to a state of being colorless and transparent.

As illustrated in Fig. 5C, the connective tissue body 21 after the solvent immersion treatment is left to stand in the drying environment as with in the atmosphere of inert gas or the like. Then, the fat seeps through the surface of the connective tissue body 21 as the connective tissue body 21 dries, and the connective tissue body 21 is deformed such that the connective tissue body 21 adheres to the outer surface of the cylindrical die 12. Accordingly, the inner diameter of the connective tissue body 21 is kept at the inner diameter when the solvent immersion treatment is ended in a portion adhered to the large-diameter portion 121. In addition, the inner diameter of the connective tissue body 21 is considerably less than the inner diameter when the solvent immersion treatment is ended in a portion adhered to the small-diameter portion 122. Then, the density of the proteins in the connective tissue body 21 increases, in particular, in the portion adhered to the small-diameter portion 122, and a two-stage tubular shape that conforms to the shape of the outer surface of the cylindrical die 12 is stored in the connective tissue body 21. At this time, the connective tissue body 21 that is close to a white color before the drying treatment is changed to the brownish yellow connective tissue body 21.

As illustrated in Fig. 5D, the connective tissue body 21 after the fat treatment, is immersed in the bioinert water such as saline, together with the cylindrical die 12. Then, the bioinert water infiltrates into the connective tissue body 21 and the thickness of the connective tissue body 21 is greater than the thickness when the fat treatment is ended in each of the portion adhered to the large-diameter portion 121 and the portion adhered to the small-diameter portion 122 so that the thickness is generally equal to the thickness before the drying treatment. During such a period, the inner diameter of the connective tissue body 21 is equal to the outer diameter Ra in the portion adhered to the large-diameter portion 121 and is equal to the outer diameter Rb in the portion adhered to the small-diameter portion 122. That is, even in the connective tissue body 21 having a large thickness, the shape of a two-stage cylindrical surface that conforms to the shape of an outer surface of the cylindrical member 11 is stored in the inner circumferential surface of the connective tissue body 21.

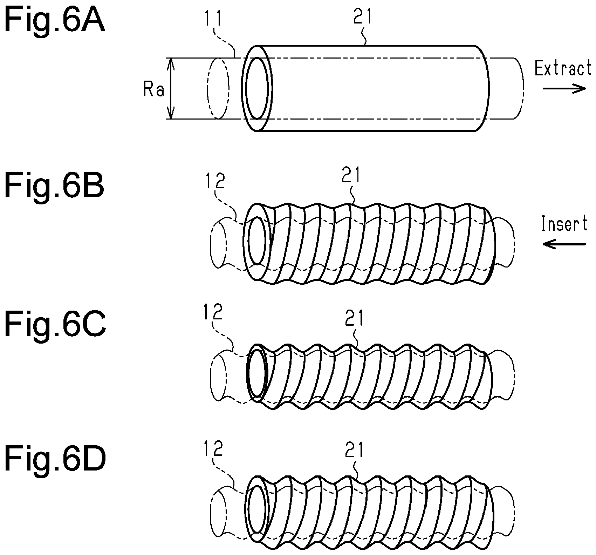

As illustrated in Fig. 6A, the cylindrical member 11 is taken out from the environment in which the biological tissue exists. The connective tissue body 21 is positioned on the outer surface of the taken cylindrical member 11. The outer surface of the cylindrical member 11 is a smooth surface and functions as the tissue body forming surface in the tissue body forming device. Then, the cylindrical member 11 is extracted from the tube of the connective tissue body 21, and a tubular connective tissue body 21 including the smooth surface as a cylinder inner circumferential surface is formed.

As illustrated in Fig. 6B, the cylindrical die 12 functioning as the molding die is set on the connective tissue body 21. An outer circumferential surface of the cylindrical die 12 is a surface including ridges and valleys forming a spiral projection extending in an extension direction of the cylindrical member 11. The connective tissue body 21 set in the cylindrical die 12 is immersed in the organic solvent such as ethanol together with the cylindrical die 12. The fat contained in the connective tissue body 21 is eluted in the organic solvent from the outer circumferential surface of the connective tissue body 21 and the inner circumferential surface of the connective tissue body 21. The immersion time in the immersion treatment, for example, is the immersion time T3 described in Fig. 2. When the solvent immersion treatment is ended, the connective tissue body 21 that is whitish before the solvent immersion treatment becomes generally colorless and transparent.

As illustrated in Fig. 6C, the connective tissue body 21 after the solvent immersion treatment is left to stand in the drying environment as with in the atmosphere of inert gas or the like. Then, the organic solvent, the moisture, or the like is removed from the inside of the connective tissue body 21 as the connective tissue body 21 dries, and the connective tissue body 21 is deformed such that the connective tissue body 21 adheres to the outer surface of the cylindrical die 12. Accordingly, the inner surface of the connective tissue body 21 is deformed from the smooth surface before the solvent immersion treatment, and becomes the surface including ridges and valleys and forming the spiral projection extending in the extension direction of the connective tissue body 21. Then, the density of the proteins in the connective tissue body 21, increases, and a tubular shape that conforms to the shape of the outer surface of the cylindrical die 12 is stored in the connective tissue body 21. At this time, the connective tissue body 21 that is colorless and transparent before the drying treatment is changed to the brownish yellow connective tissue body 21.

As illustrated in Fig. 6D, after the fat treatment, the connective tissue body 21 is immersed in the bioinert water such as saline together with the cylindrical die 12. Then, the bioinert water infiltrates into the connective tissue body 21, and the thickness of the connective tissue body 21 is slightly greater than the thickness when the drying treatment is ended. During such a period, the inner circumferential surface of the connective tissue body 21 is kept at the shape that conforms to the shape of the outer circumferential surface of the cylindrical die 12. As a result, it is possible to store the shape that conforms to the shape of the outer surface of the cylindrical die 12 in the connective tissue body 21, and to broaden the range with respect to the design value on the structure connective tissue body 21. Then, the connective tissue body 21 and the cylindrical die 12 are relatively rotated from such a state thereby separating the connective tissue body 21 and the cylindrical die 12 are separated from each other.

As illustrated in Fig. 7A, a cylindrical member 13 is taken out from the environment in which the biological tissue exists. A connective tissue body 22 is positioned on the outer surface of the taken cylindrical member 13. The cylindrical member 13 includes successive folded portions in the extension direction of the cylindrical member 13. The cylindrical member 13 is flexible and functions as a tissue body forming device that resists elastic deformation. In addition, the cylindrical member 13 functions as a straight pipe die 14 that is an example of the molding die in the setting step, the fat treatment step, and the bioinert water treatment step described above.

As illustrated in Fig. 7B, in the straight pipe die 14, the folded portion linearly extends, in a state where the connective tissue body 22 is positioned on the outer surface of the straight pipe die 14. That is, the straight pipe die 14 and the connective tissue body 22 are processed into a single straight line. Accordingly, the connective tissue body 22 is set in the straight pipe die 14 functioning as the molding die. Next, the connective tissue body 22 set in the straight pipe die 14 is immersed in the organic solvent such as ethanol together with the straight pipe die 14 as the fat treatment. Accordingly, the fat contained in the connective tissue body 22 is eluted in the organic solvent. The immersion time in the solvent immersion treatment, for example, is the retention time T1 described in Fig. 2. When the fat treatment is ended, the connective tissue body 22 that is whitish before the fat treatment becomes closer to a state of being colorless and transparent than before.

After the fat treatment, the connective tissue body 22 is immersed in the bioinert water such as saline together with the straight pipe die 14, and the bioinert water infiltrates the connective tissue body 22. When the bioinert water treatment is ended, the thickness of the connective tissue body 22 is generally equal to the thickness of the connective tissue body before the fat treatment. During such a period, an extension direction of the connective tissue body 22 is kept as a single linear direction that conforms to an extension direction of the straight pipe die 14. As a result, as illustrated in Fig. 7C, it is possible to store a linear shape that conforms to the extension direction of the straight pipe die 14 in the connective tissue body 22 and to broaden the range with respect to the design value on the structure of the connective tissue body 22.

As illustrated in Fig. 8A, the cylindrical member 11 is taken out from the environment in which the biological tissue exists. A connective tissue body 23 is positioned on the outer surface of the taken cylindrical member 11. The cylindrical member 11 functions as the tissue body forming device. Then, the cylindrical member 11 is extracted from the tube of the connective tissue body 23, and a tubular connective tissue body 23 is formed.

As illustrated in Fig. 8B, a part of the connective tissue body 23 is cut along the extension direction of the connective tissue body 23, and thus, the tubular connective tissue body 23 is spread into the shape of a sheet. The connective tissue body 23, which has been spread into the shape of a sheet, is set on a molding surface of a plate-shaped die 15 functioning as the molding die in a spread-out state. Next, the connective tissue body 23, which is set in the plate-shaped die 15, is left to stand in the drying environment as with in the atmosphere of inert gas or the like as the fat treatment. Accordingly, the fat contained in the connective tissue body 23 seeps through the surface of the connective tissue body 23 and decreases the thickness of the connective tissue body 23.

As illustrated in Fig. 8C, after the fat treatment, the connective tissue body 23 is immersed in the bioinert water such as saline together with the plate-shaped die 15. Then, the bioinert water infiltrates into the connective tissue body 23, and the thickness of the connective tissue body 23 is slightly greater than the thickness when the fat treatment is ended. During such a period, the shape of the connective tissue body 23 is kept to the shape of a flat plate that conforms to the shape of the molding surface of the plate-shaped die 15. That is, even in the connective tissue body 23 having a large thickness, the shape that conforms to the shape of the molding surface of the plate-shaped die 15 is stored. As a result, it is possible to store the shape that conforms to the shape of the molding surface of the plate-shaped die 15 in the connective tissue body 23 and to broaden the range with respect to the design value on the structure of the connective tissue body 23.

As described above, according to the embodiment described above, it is possible to obtain the following advantages.
(1) It is possible to broaden the range with respect to the design value on the structure such as the dimension or the shape of the connective tissue body.
(2) For example, it is possible to decrease the thickness of the connective tissue body, decrease the inner diameter of the connective tissue body, have the surface shape of the connective tissue body a multi-stage shape, have the surface shape of the connective tissue body include ridges and valleys, and have the connective tissue body shaped as a flat plate.
(3) The density of the proteins in the extracellular matrix of the connective tissue body is increased thereby increasing the mechanical strength of the connective tissue body used in the regeneration medicine.

It will be apparent to those skilled in the art from this disclosure that the embodiment described above may be modified as follows.

In each of the tissue body forming device and the molding die, for example, a groove for aligning the proteins in a direction intersecting with a direction in which the mechanical strength of the connective tissue body is required to increase can be provided on a surface with which the connective tissue body is in contact. For example, in the cylindrical members 11 and 13, when increasing the mechanical strength in a circumferential direction of the connective tissue body, a fine groove extending in the extension direction of the cylindrical members 11 and 13 is provided on the outer circumferential surface of the cylindrical members 11 and 13. In addition, for example, in the cylindrical die 12 and the straight pipe die 14, in the case of increasing the mechanical strength in the circumferential direction of the connective tissue body, a fine groove extending in the extension direction of the cylindrical die 12 and the straight pipe die 14 is provided on the outer circumferential surface of the cylindrical die 12 and the straight pipe die 14. According to each of the tissue body forming device and the molding die, as described above, it is possible to increase the strength of the connective tissue body in a desired direction of the connective tissue body.

It should be apparent to those skilled in the art that the present invention may be embodied in many other specific forms without departing from the spirit or scope of the invention. For example, a part of the components described in the embodiment (or one or a plurality of aspects thereof) may be omitted and several components may be combined. The scope of the present invention is to be determined within the scope and equivalence of the appended claims.

### DESCRIPTION OF REFERENCE CHARACTERS

- Ra, Rb: Outer diameter
- Ta, Tb, Tc: Thickness
- 11, 13: Cylindrical member
- 12: Cylindrical die (molding die)
- 14: Straight pipe die (molding die)
- 15: Plate-shaped die (molding die)
- 21, 22, 23: Connective tissue body

## Claims

1. A method for forming a connective tissue body, the method comprising:
a fat treatment step of removing fat contained in a connective tissue body, which is formed in an environment in which a biological tissue material exists and set in a molding die, from an inside of the connective tissue body and shaping the connective tissue body in conformance with a shape of the molding die; and
a bioinert water treatment step of immersing the connective tissue body in bioinert water along with the molding die in a state in which the shape of the connective tissue body after the fat treatment step conforms to the shape of the molding die.

2. The method for forming a connective tissue body according to claim 1, wherein the removing the fat from the inside of the connective tissue body includes eluting the fat from the connective tissue body with an organic solvent that dissolves the fat by immersing the connective tissue body in the organic solvent in a state set in the molding die.

3. The method for forming a connective tissue body according to claim 1 or 2, wherein the removing the fat from the inside of the connective tissue body includes seeping the fat contained in the connective tissue body through a surface of the connective tissue body, by drying the connective tissue body in a state set in the molding die.

4. The method for forming a connective tissue body according to claim 1, wherein the removing the fat from the inside of the connective tissue body includes eluting the fat from the connective tissue body in an organic solvent by immersing the connective tissue body in a state set in the molding die and drying the connective tissue body after being immersed in the organic solvent together with the molding die.

5. The method for forming a connective tissue body according to any one of claims 1 to 4, wherein the bioinert water treatment step keeps a density of proteins in an extracellular matrix of the connective tissue body higher than that before the fat treatment step.

6. The method for forming a connective tissue body according to claim 3 or 4, wherein
the fat treatment step thins the connective tissue body thin through drying, and
the bioinert water treatment step sets a thickness of the connective tissue body to be greater than the thickness after the drying and less than the thickness before the fat treatment step.

7. The method for forming a connective tissue body according to claim 4, wherein the fat treatment step elutes the fat from the connective tissue body in the organic solvent by immersing the connective tissue body in the organic solvent in a state in which the molding die having a smaller diameter than a tubular portion of the connective tissue body is internally inserted into the connective tissue body to closely attach the tubular portion to the molding die through the drying.

8. The method for forming a connective tissue body according to claim 2, wherein the fat treatment step includes eluting the fat from the connective tissue body in the organic solvent by immersing the connective tissue body in the organic solvent after the molding die, which extends in a direction differing from an extension direction of a tubular portion of the connective tissue body, is internally inserted into the connective tissue body and an extension direction of the connective tissue body conforms to an extension direction of the molding die.
